(19) **Europäisches Patentamt**

**European Patent Office**

**Office européen des brevets**

(11) **EP 1 097 711 B1**

(12) **EUROPEAN PATENT SPECIFICATION**

(45) Date of publication and mention
of the grant of the patent:
**17.05.2006 Bulletin 2006/20**

(51) Int Cl.:
*A61K 31/519* (2006.01)     *A61K 31/496* (2006.01)
*A61K 31/53* (2006.01)      *A61K 31/501* (2006.01)
*A61K 31/517* (2006.01)     *A61P 11/00* (2006.01)

(21) Application number: **00309212.9**

(22) Date of filing: **01.11.2000**

(54) **Treatment of pulmonary hypertension**

Behandlung der pulmonalen Hypertension

Traitement de l'hypertension pulmonaire

(84) Designated Contracting States:
**AT BE CH CY DE DK ES FI FR GB GR IE IT LI LU
NL PT SE TR**

(30) Priority: **02.11.1999 GB 9925970
11.02.2000 GB 0003235**

(43) Date of publication of application:
**09.05.2001 Bulletin 2001/19**

(73) Proprietors:
• **Pfizer Limited
Sandwich, Kent CT13 9NJ (GB)**
Designated Contracting States:
**GB**
• **PFIZER INC.
New York, N.Y. 10017 (US)**
Designated Contracting States:
**AT BE CH CY DE DK ES FI FR GR IE IT LI LU NL
PT SE TR**

(72) Inventors:
• **Butrous, Ghazwan Saleem
Sandwich,
Kent, CT13 9NJ (GB)**
• **Lukas, Timothy Michael
Sandwich,
Kent, CT13 9NJ (GB)**
• **Machin, Ian
Sandwich,
Kent, CT13 9NJ (GB)**

(74) Representative: **McMunn, Watson Palmer
Pfizer Limited
Patents Department
Ramsgate Road
Sandwich, Kent CT13 9NJ (GB)**

(56) References cited:
**WO-A-95/19978          WO-A-98/49166
WO-A-99/54333**

• **ATZ, ANDREW M. ET AL: "Sildenafil ameliorates
effects of inhaled nitric oxide withdrawal."
ANESTHESIOLOGY (HAGERSTOWN), (JULY,
1999) VOL. 91, NO. 1, PP. 307-310., XP000991135**
• **BIGATELLO L.M. ET AL: "Strategies to enhance
the efficacy of nitric oxide therapy."
RESPIRATORY CARE, (1999) 44/3 (331-339).,
XP000991124**
• **ABRAMS D ET AL: "Sildenafil as a selective
pulmonary vasodilator in childhood primary
pulmonary hypertension." HEART (BR CARD
SOC ONLINE), (2000 AUG) 84 (2) E4.,
XP000992147**
• **BLAND R.D.: "Inhaled nitric oxide: A premature
remedy for chronic lung disease?." PEDIATRICS,
(1999) 103/3 (667-670)., XP000991122**
• **PRASAD S. ET AL: "Sildenafil in primary
pulmonary hypertension [5]." NEW ENGLAND
JOURNAL OF MEDICINE, (2 NOV 2000) 343/18
(1342)., XP000991169**

Remarks:
The file contains technical information submitted after
the application was filed and not included in this
specification

Note: Within nine months from the publication of the mention of the grant of the European patent, any person may give
notice to the European Patent Office of opposition to the European patent granted. Notice of opposition shall be filed in
a written reasoned statement. It shall not be deemed to have been filed until the opposition fee has been paid. (Art.
99(1) European Patent Convention).

**Description**

This invention relates to the use of sildenafil for the treatment of pulmonary hypertension selected from primary pulmonary hypertension and secondary pulmonary hypertension, wherein the secondary pulmonary hypertension is secondary to congestive heart failure, chronic hypoxic lung disorder, inflammatory or collagen vascular disease, congenital heart disease associated with left to right shunting, and pulmonary thromboembolism

According to the specification of our International patent application WO94/28902 we have discovered that compounds which are inhibitors of the cGMP PDE5 enzyme are potent and effective compounds for the treatment of male erectile dysfunction (MED, impotence) and for female sexual disorders. This discovery led to the development of the compound sildenafil (5-[2-ethoxy-5-(4-methyl-1-piperazinylsulphonyl)phenyl]-1-methyl-3-n-propyl-1,6-dihydro-7H-pyrazolo[4,3-d]pyrimidin-7-one) (VIAGRA™) which has proved to be outstandingly successful as the first orally effective treatment for MED.

Pulmonary hypertension is a pathological condition in which the pulmonary arterial pressure rises above normal levels and may cause sequelae of haemodynamic changes that can become life threatening. Symptoms of pulmonary hypertension include shortness of breath with minimal exertion, fatigue, dizzy spells and fainting. When pulmonary hypertension occurs in the absence of a known cause, it is referred to as primary pulmonary hypertension. Primary pulmonary hypertension is rare, occurring in about two per million people worldwide.

Secondary pulmonary hypertension is much more common occurring as a result of other medical conditions, including congestive heart failure, chronic hypoxic lung disorder, including chronic obstructive pulmonary disease, inflammatory or collagen vascular diseases such as scleroderma and systemic lupus erythematosus, congenital heart diseases associated with left to right shunting and pulmonary thromboembolism.

Since pulmonary hypertension is caused typically by constriction of the pulmonary blood vessels, vascular resistance is the favoured indicator of the disease. The pulmonary vascular resistance (PVR) and systemic vascular resistance (SVR) are calculated as follows.

$$PVR = \frac{\text{(mean pulmonary artery pressure-pulmonary wedge pressure)} \times 80}{\text{cardiac output}}$$

$$SVR = \frac{\text{(mean arterial blood pressure- systemic venous pressure)} \times 80}{\text{cardiac output}}$$

Agents which selectively lower PVR without significant lowering SVR remain limited.

The use of phosphodiesterase inhibitors administered endotracheally or endobronchially to treat pulmonary hypertension has been described in WO95/09636 but the compounds employed were neither particularly potent nor selective cGMP PDE inhibitors.

Atz et al (Anesthesiology (July 1999) Vol. 91, No 1, pp. 307-310) describes the use of sildenafil to potentiate the effects of No and overcome rebound pulmonary hypertension in post-operative patients.

WO 98/37894 describes the use of a PDE inhibitor and an adenylate cyclase agonist or a guanylate cyclase agonist in the treatment of disease states which are based on acute or chronic destruction of vessels and/or bronchi, acute or chronic inflammation and/or edema formation.

The invention provides for the use of sildenafil or a pharmaceutically acceptable salt, preferably sildenafil citrate solvate or polymorph thereof; for the manufacture of a medicament for treating or preventing pulmonary hypertension as defined in Claim 1.

For the preparation of sildenafil, (5-[2-ethoxy-5-(4-methylpiperazin-1-ylsulfonyl)phenyl]-1,6-dihydro-1-methyl-3-propylpyrazolo[4,3-d]pyrimidin-7-one) see Example 12 of EP 0463756.

Hereinafter, sildenafil is referred to as the compound of the invention, and includes pharmaceutical salts, solvates or polymorphs thereof.

Advantageously, we have shown that the compound of the invention lowers the PVR to a greater extent than SVR.

Compound of the invention can be administered alone but, in human therapy will preferably be administered in admixture with a suitable pharmaceutical excipient, diluent or carrier selected with regard to the intended route of administration and standard pharmaceutical practice.

Compound of the invention can be administered orally, buccally or sublingually in the form of tablets, capsules, ovules, elixirs, solutions or suspensions, which may contain flavouring or colouring agents, for immediate-, delayed-, modified-, or controlled-release applications. Tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethyl cellulose, hydroxypropylcellulose, sucrose, gelatin and acacia. Additionally, lubricating agents such as magnesium stearate, stearic acid, glyceryl behenate and talc may be included. Excipients of a similar type may also be employed as fillers in gelatin capsules. Preferred excipients in this regard include lactose, starch, a cellulose, milk sugar or high molecular weight polyethylene glycols. For aqueous suspensions and/or elixirs, compounds of the invention may be combined with various sweetening or flavouring agents, colouring matter or dyes, with emulsifying and/or suspending agents and with diluents such as water, ethanol, propylene glycol and glycerin, and combinations thereof.

In humans, oral administration of the compound of the invention is a preferred route. In circumstances where the recipient suffers from a swallowing disorder or from impairment of drug absorption after oral administration, the drug may be administered parenterally, sublingually or buccally.

The compound of the invention can also be administered parenterally, for example, intracavernosally, intravenously, intra-arterially, intraperitoneally, intrathecally, intraventricularly, intraurethrally intrasternally, intracranially, intramuscularly or subcutaneously, or they may be administered by infusion techniques. For such parenteral administration they are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

Compound of the invention can also be administered intranasally or by inhalation. Inhaled formulations have advantages in delivering the active compound directly to the lung area, producing a faster effect than orally delivered formulations. For this embodiment the aerosol particle size is preferably between 0.5 micrometers and 5 micrometers. The aerosol is conveniently generated from a pressurised container, pump, spray or nebuliser with the use of a suitable propellant, e.g dichlorodifluoromethane, trichlorofluoromethane, dichlorotetrafluoroethane, a hydrofluoroalkane such as 1,1,1,2-tetrafluoroethane or 1,1,1,2,3,3,3-heptafluoropropane, carbon dioxide or other suitable gas. In the case of a pressurised aerosol, the dosage unit may be determined by providing a valve to deliver a metered amount. In a preferred embodiment, the compound of the invention are administered by inhalation.

The term inhalation or inhaled includes endotracheal and endobronchial administration.

Solutions of the active ingredient for use in such inhalers are prepared by conventional methods, typically by dissolving the active ingredient in water which is preferably buffered to pH 3-8, more preferably pH 4 to 7 using standard buffer systems such as citrate, lactate or phosphate buffers to control the pH. Ethanol may also be added at a concentration of up to 30% to improve aerosolisation of the formulation. Additional stabilisers may be required to improve chemical stability of the formulations; ie antioxidants, such as sodium metabisulphite, sodium bisulphite or tocopherol, or metal chelators such as ethylenediaminetetraacetic acid.

Single unit-dose spray can be prepared aseptically or terminally sterilised to produce a sterile final product. Alternatively, multi-dose metered nebulisers, inhalers or atomisers can be used.

Flavourings, perfumes and humectants may also be added to improve the patient acceptability of the formulation. Solubility enhancers e.g. caffeine can be added to improve solubility of the active drug.

For oral and parenteral administration to human patients, the daily dosage level of the compound of the invention will usually be less than 500 mg (in single or divided doses). Thus, for example, tablets or capsules of sildenafil may contain less than 50 mg of active compound for administration singly or two or more at a time, as appropriate. The physician in any event will determine the actual dosage which will be most suitable for any individual patient and it will vary with the age, weight and response of the particular patient. The above dosages are exemplary of the average case. There can, of course, be individual instances where higher or lower dosage ranges are merited and such are within the scope of this invention. The skilled person will also appreciate that, in the treatment of certain conditions, compound of the invention may be taken as a single dose on an "as required" basis (i.e. as needed or desired).

We have shown during studies on dog models, that pulmonary blood vessels are more sensitive to the actions of the compounds of the invention than is the *corpus cavernosum*. In the *corpus cavernosum* of the penis, a calculated intravenous dose of 12 microgrammes/kg sildenafil produced a half-maximal potentiation ($ED_{50}$) of nerve-induced pressure rises (see Carter A J, Ballard S A and Naylor A M, *The Journal of Urology*, (1998), volume 160, pages 242-246). In contrast a dose of only 1.5 microgrammes/kg produced a maximal response in terms of reversing hypoxic pulmonary vasoconstriction (see Biological Studies hereinafter). This is surprising in that both effects are thought to be mediated via the inhibition of PDE5. Accordingly a preferred dose of the compound of the invention for treating pulmonary hypertension is up to 50 mg, more preferably up to 20 mg, more preferably up to 10 mg, more preferably 1 to 10 mg. Administration of such low doses to a patient significantly reduces the risk of side effects.

The preferred PDE5 inhibitor for oral administration is sildenafil (preferably sildenafil citrate), in dosages of up to 50 mg, more preferably up to 20 mg, more preferably up to 10 mg, more preferably 1 to 10 mg.

The citrate salt of sildenafil is the preferred salt for oral administration, however other pharmaceutically acceptable salts may also be used.

Alternatively the drug may be administered as a micronised powder. The drug is micronised to give a particle size in the range 0.1 to 5 micrometres, preferably less than 1 micrometre, and then blended with a suitable lactose carrier. The powder can be placed in hard gelatin capsules for use in conjunction with a conventional dry powder inhalation device.

The inhaled formulations described above should deliver a dose of a compound of the invention of up to 50 mg, more preferably up to 20 mg, more preferably up to 10 mg, more preferably 1 to 10 mg. The exact dose administered will, however, differ depending on the subject being treated, on the severity of the condition, on the manner of administration and on the judgment of the prescribing physician. Thus, because of patient-to-patient variability, the dosages given below are a guideline only and the physician may adjust doses of the compounds to achieve the treatment that the physician considers appropriate for the patient. In considering the degree of treatment desired, the physician must balance a variety of factors such as the age of the patient and the presence of other diseases or conditions (e.g. cardiovascular disease).

The preferred PDE5 inhibitor for inhaled administration is sildenafil, in dosages of up to 50 mg, more preferably up to 20 mg, more preferably up to 10 mg, more preferably 1 to 10 mg.

A preferred formulation for administration by inhalation comprises an aqueous formulation of sildenafil mesylate for use in an aerosol nebuliser or atomiser to provide a dose of less than 20 mg of sildenafil mesvlate per dose.

The compound used according to the invention can also be administered together with prostacyclins (e.g. Epoprostenol), oxygen, calcium channel blockers (e.g. Nifedipine, Diltazem, Amlodipine), endothelin antagonists (ETa), iloprost, adensosine and/or nitric oxide.

In addition to treatment of adult patients, a further application of the invention is in the treatment of very young children born with congenital heart disease. Compound of the invention can be used to treat pulmonary hypertension in such subjects and can thus delay the immediate need for surgery until the patient is better able to withstand the trauma of surgery. Compound of the invention can also be used to treat children who have respiratory distress syndrome or neonatal hypoxia.

Alternatively, compound of the invention can be administered in the form of a suppository or pessary, or they may be applied topically in the form of a gel, hydrogel, lotion, solution, cream, ointment or dusting powder. Compound of the invention may also be dermally or transdermally administered, for example, by the use of a skin patch. They may also be administered by the ocular, pulmonary or rectal routes.

Compound of the invention may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and be dermally or transdermally administered, for example, by the use of a skin patch. They may also be administered by the ocular, pulmonary or rectal routes.

Compound of the invention may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and gamma-cyclodextrins are most commonly used and suitable examples are described in WO-A-91/11172, WO-A-94/02518 and WO-A-98/55148.

It is to be appreciated that all references herein to treatment include curative, palliative and prophylactic treatment.

Methods of preparing such pharmaceutical compositions with a certain amount of active ingredient are well known to those skilled in this art, or may be determined by reference to literature precedents.

Examples of particular formulations are included hereinafter to further illustrate the invention.

Example 1

A Tablet Formulation for Oral Use

Sildenafil citrate (20 mg) is blended with cellulose (microcrystalline), silicon dioxide, stearic acid (fumed) and the mixture is compressed to form tablets.

Example 2

An Intravenous Formulation

| Sildenafil citrate | 100mg |
|---|---|
| isotonic saline | 1,000ml |

Example 3

Dry Powder Formulation for Inhalation

A dry powder formulation of sildenafil citrate was prepared by blending micronised drug (1 g) with lactose suitable for inhalation use, e.g. Pharmatose (trade mark), 325 mesh, (10 g) to provide a homogeneous blend. The product was filled into hard gelatin capsules (150 mg) for use with a commercial dry powder inhalation device.
Similar formulations may be prepared of sildenafil mesylate and sildenafil free base.

Example 4

Solution Formulation for Inhalation

A solution was prepared of sildenafil mesylate having the following composition:

| | |
|---|---|
| Sildenafil mesylate | 10g |
| Sodium dihydrogen phosphate | 0.69g |
| Distilled water | 90ml |
| Ethanol | 10ml |

The solution was stirred to dissolve the ingredients and the pH adjusted to 4.2 by the addition of 1M sodium hydroxide solution. The solution was filter sterilised and aseptically filled into amber nebuliser bottles. This solution may then be used with commercial nebulisers or atomisers to dose 10 mg of active drug.

Preparation of 5-[2-ethoxy-5-(4-methylpiperazin-1-ylsulphonyl)phenyl]-1,6-dihydro- 1-methyl- 3-propylpyrazolo [4,3-d] pyrimidin-7-one) methanesulphonate salt (sildenafil mesylate)

5-[2-Ethoxy-5-(4-methylpiperazin-1-ylsulphonyl)phenyl]-1, 6-dihydro-1-methyl-3-propylpyrazolo[4,3-d]pyrimidin-7-one (sildenafil, see EP 0463756 Example 12 for preparation) (100g, 0.21 mol) was dissolved in boiling acetone (3000 ml). Methanesulphonic acid (14.9 ml, 0.23 mol) was added to the hot acetone solution. Within 10 seconds a precipitate formed. The mixture was allowed to cool and granulate for 48 hours. The title product was collected by filtration and dried in vacuum to give a white crystalline solid (116.0 g, 96.8%), m.p. 272-274°C; Found: C, 48.33; H, 5.99; N, 14.68. $C_{23}H_{34}N_6O_7S_2$ requires C, 48.41; H, 6.00; N, 14.73%; $\delta(CD_3SOCD_3)$ 0.92 (3H, t), 1.33 (3H, t), 1.73 (2H, heptet), 2.29 (3H,s), 2.77 (2H, t), 2.79 (3H, s), 3.16 (2H, br), 3.3-3.57 (4H, br), 3.8 (2H, br), 4.16 (3H, s), 4.20(2H, q), 7.4 (1H, d), 7.88 (1H, dd), 7.90 (1H, s) and 9.44 (1H, br).

Clinical Study

The efficacy of sildenafil in pulmonary hypertension in human patients was demonstrated by the following study.
A number of patients having various causes of pulmonary hypertension were selected. The patients were then assessed in order to establish baseline data, including haemodynamic parameters (by right heart catheterisation), blood gas profile (via an arterial line and pulse oximetry). The patients were then tested with 40 ppm of nitric oxide (NO) by inhalation for 5 minutes to assess the reversibility of pulmonary hypertenion. Haemondynamic parameters were reassessed within 5 minutes of NO and then further assessed 5 -20 minutes later. When the pulmonary arterial pressure levels had returned to baseline (+-5%) inhalation haemodynamic parameters were assessed again and patients were continuously infused with sildenafil at rates to control the plasma level at 100, 300 and 500 nanogrammes per millilitre. A few patients received placebo instead of sildenafil in double-blind fashion. Haemodynamic parameters were recorded throughout infusion.

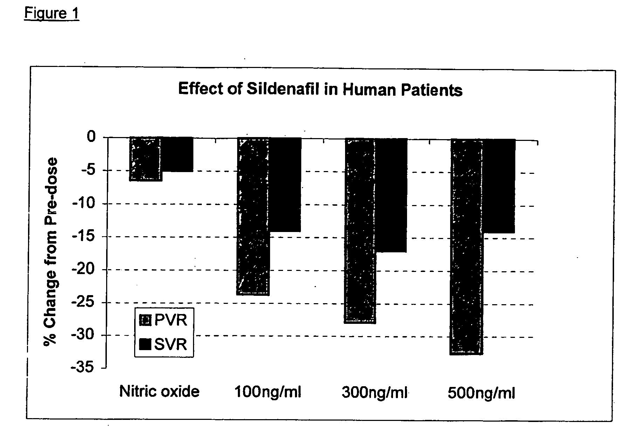

From the data collected during the trial, the PVR and SVR were determined. The results are shown in Figure 1 and demonstrate a significant reduction in PVR experienced in a number of patients, confirming the utility of sildenafil for this indication. Furthermore, the results demonstrate that the effect of sildenafil on the SVR was substantially lower than the effect on PVR.

Biological Studies

The efficacy of sildenafil for treating pulmonary hypertension in dogs was demonstrated by the following studies. The first study examined efficacy via intravenous administration. The second study examined efficacy via inhaled administration.

*Intravenous Administration*

The anaesthetised dog is the model of choice in which to study the effects of drugs, owing to the similarity between canine and human haemodynamics, and additionally, to ensure consistency between pharmacological and toxicological species.

Beagle dogs of either sex were anaesthetised with intravenous sodium pentobarbitone (Sagatal [trademark], 30 to 45 mg/kg) and anaesthesia maintained with an infusion of Sagatal (6 mg/kg/h) into the right femoral vein. The dogs were initially intubated via the mouth, and then via a tracheotomy, and artificially respired using an Ugo Basille dog respirator. If necessary respiratory reflexes were abolished by the administration of pancuronium (0.2 mg/kg, at 60 to 90 minute intervals). Respiratory gases (inspired $O_2$ and end tidal $CO_2$) were monitored by a Normocap 200 (trademark) respiratory gas monitor (Datex Instrumentarium Corp.). The depth of anaesthesia was assessed by carefully monitoring arterial blood pressure and heart rate. The left femoral vein and artery were cannulated for dose administration and recording arterial blood pressure, respectively. The right jugular vein was cannulated with a Swan Ganz catheter, the tip of which was positioned by means of pressure monitoring in a branch of the pulmonary artery. Pulmonary artery pressure (PAP) and pulmonary artery wedge pressure were recorded from this catheter. This catheter was also connected to a cardiac index computer (Gould Instruments) and cardiac output determined by thermodilution following rapid injection of 5 ml saline at room temperature. All catheters were filled with heparinised saline. Lead II ECG was recorded from 2 stainless steel needles inserted subcutaneously in the right arm and left leg. All transducers and recording electrodes were connected to HSE preamplifiers. All data from the primary signals (PAP, Arterial blood pressure (ABP) and ECG) was recorded on a Po-ne-mah (trademark) data acquisition system. From these signals the following parameters were derived; systolic, diastolic and mean blood pressure, systolic, diastolic and mean pulmonary artery pressure, pulmonary capillary wedge pressure. Heart rate was recorded from both the blood pressure and ECG signals, for verification checks of the analysis algorithms.

Arterial blood samples were taken at intervals to monitor blood gases using an ABL505 blood gas analyser (Radiometer Ltd.). Throughout the experiment Hartmans solution was infused at a rate appropriate to maintain blood acid/base balance and volume. Core temperature was maintained at approximately 38°C by means of a Harvard Homeothermic blanket. Following preparatory surgery, the animals were allowed to stabilise for approximately 30 minutes.

The haemodynamic parameters were logged by the data acquisition system as the mean values from complete cardiac cycles every 10 seconds. The computer file containing these values constitutes the raw data for the study. During the control periods inspired oxygen was at a concentration of 40%. Hypoxia was produced by adding nitrogen to the inspiratory gas mixture at a rate sufficient to reduce inspired oxygen to 10%. Hypoxic conditions were maintained for 15 minutes during which time cardiac output estimations were taken at 5 minute intervals. Following this hypoxic challenge the dog was returned to control conditions for 30 minutes before the next challenge. Three baseline measurements of all parameters were taken at 5 minute intervals prior to the animals receiving an hypoxic challenge. These baseline readings check the stability of the preparation. Two hypoxic challenges were conducted before the addition of any compound or vehicle.

Each intravenous dose of sildenafil consisted of a 2 minute loading infusion starting 15 minutes before an hypoxic challenge, followed by a maintenance infusion which continued throughout the hypoxic challenge and subsequent normoxia until the next dose is given. Vehicle was given at the same infusion rates as those used for sildenafil and the rate of the infusions did not exceed 2 ml/minute. A 2 ml sample of arterial blood was taken at pre dose and just prior to the end of each infusion, the plasma frozen and the drug content was analysed.

Doses were made up by dissolving sildenafil in vehicle [sodium acetate buffer (0.038 M)] to the required concentration. Four doses of 1.5, 4.5, 15 and 45 microgrammes/weight of dog in kilogrammes were administered.

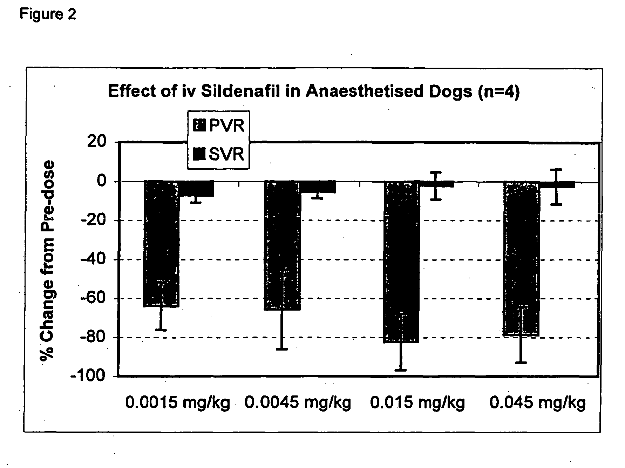

The results are shown in Figure 2, which shows the effect on PVR and SVR against dose. The results show a maximal effect on the PVR at the lowest dose of 1.5 microgrammes/kg. The effect on SVP is significantly lower than on PVP.

*Inhaled Administration*

This separate study was performed in analogous fashion to intravenous administration, except that doses were administered into the inspiratory limb of the respiratory circuit using a Penta-sonic Nebuliser (deVilbiss). Each inhaled dose consisted of a timed nebulisation of the solution at a respiration rate of 15 breaths/min finishing 5 min before an hypoxic challenge. Sildenafil and vehicle were administered to a maximum of four dogs per group (i.e. total of eight dogs). At the end of the first vehicle experiment an additional hypoxic challenge was done following administration of an inhaled dose of sildenafil.

Doses were made up by dissolving sildenafil in vehicle [sodium acetate buffer (0.038 M)] to the required concentration. Four doses of 30, 80, 230 and 770 microgrammes/weight of dog in kilogrammes were administered.

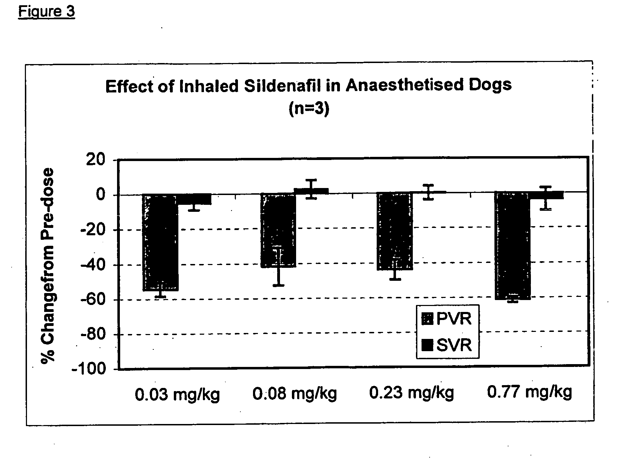

The results are shown in Figure 3, which shows the effect of dose against PVR and SVR. The results show a maximal effect on the PVP at the lowest dose of 30 microgrammes/kg.

**Claims**

1. The use of sildenafil in the manufacture of a medicament for treating or preventing pulmonary hypertension selected from primary pulmonary hypertension and secondary pulmonary hypertension, wherein the secondary pulmonary hypertension is secondary to congestive heart failure, chronic hypoxic lung disorder, inflammatory or collagen vascular disease, congenital heart disease associated with left to right shunting, and pulmonary thromboembolism, and wherein the pulmonary vascular resistance is lowered to a greater extent than the systemic vascular resistance.

2. The use according to claim 1, wherein the effective amount is less than 50 mg per day.

3. The use according to claim 2 wherein the effective amount is up to 20 mg per day.

4. The use according to claim 3 wherein the effective amount is up to 10 mg per day.

5. The use according to claim 4 wherein the effective amount is from 1 to 10 mg per day.

6. The use according to any one of claims 1 to 5 wherein the PDE5 inhibitor is administered orally.

7. The use according to claim 6 wherein the PDE5 inhibitor is sildenafil citrate.

8. The use according to any one of claims 1 to 5 wherein the PDE5 inhibitor is inhaled.

9. The use according to claim 8 wherein the PDE5 inhibitor is sildenafil mesylate.

**Patentansprüche**

1. Verwendung von Sildenafil zur Herstellung eines Arzneimittels zur Behandlung oder Prävention von Lungenhochdruck, ausgewählt aus primärem Lungenhochdruck und sekundärem Lungenhochdruck, wobei der sekundäre Lungenhochdruck sekundär ist zu einer kongestiven Herzinsuffizienz, einer chronischen hypoxämischen Lungenerkrankung, einer entzündlichen oder Kollagen-Gefäßerkrankung, einer angeborenen Herzkrankheit, die mit einem Links-Rechts-Shunt einhergeht, und einer Lungenthromboembolie, wobei der Lungengefäßwiderstand in einem größeren Ausmaß erniedrigt ist als der systemische Gefäßwiderstand.

2. Verwendung nach Anspruch 1, wobei die wirksame Menge weniger als 50 mg pro Tag beträgt.

3. Verwendung nach Anspruch 2, wobei die wirksame Menge bis zu 20 mg pro Tag beträgt.

4. Verwendung nach Anspruch 3, wobei die wirksame Menge bis zu 10 mg pro Tag beträgt.

5. Verwendung nach Anspruch 4, wobei die wirksame Menge 1 bis 10 mg pro Tag beträgt.

6. Verwendung nach einem der Ansprüche 1 bis 5, wobei der PDE5-Inhibitor oral verabreicht wird.

**7.** Verwendung nach Anspruch 6, wobei der PDE5-Inhibitor Sildenafilcitrat ist.

**8.** Verwendung nach einem der Ansprüche 1 bis 5, wobei der PDE5-Inhibitor inhaliert wird.

**9.** Verwendung nach Anspruch 8, wobei der PDE5-Inhibitor Sildenafilmesylat ist.

**Revendications**

**1.** Utilisation de sildénafil dans la fabrication d'un médicament destiné au traitement ou à la prévention d'une hypertension artérielle pulmonaire choisie parmi l'hypertension artérielle pulmonaire primitive et l'hypertension artérielle pulmonaire secondaire, où l'hypertension artérielle pulmonaire secondaire est secondaire à une insuffisance cardiaque, un trouble pulmonaire hypoxique chronique, une maladie vasculaire inflammatoire ou du collagène, une cardiopathie congénitale associée à un shunt gauche-droite, et une thromboembolie pulmonaire, et où la résistance vasculaire pulmonaire est abaissée dans une plus grande mesure que la résistance vasculaire systémique.

**2.** Utilisation selon la revendication 1, dans laquelle la dose efficace est inférieure à 50 mg par jour.

**3.** Utilisation selon la revendication 2, dans laquelle la dose efficace est d'au plus 20 mg par jour.

**4.** Utilisation selon la revendication 3, dans laquelle la dose efficace est d'au plus 10 mg par jour.

**5.** Utilisation selon la revendication 4, dans laquelle la dose efficace est de 1 à 10 mg par jour.

**6.** Utilisation selon l'une quelconque des revendications 1 à 5, dans laquelle l'inhibiteur de PDE5 est administré par voie orale.

**7.** Utilisation selon la revendication 6, dans laquelle l'inhibiteur de PDE5 est le citrate de sildénafil.

**8.** Utilisation selon l'une quelconque des revendications 1 à 5, dans laquelle l'inhibiteur de PDE5 est inhalé.

**9.** Utilisation selon la revendication 8, dans laquelle l'inhibiteur de PDE5 est le mésylate de sildénafil.

Figure 1

Effect of Sildenafil in Human Patients

% Change from Pre-dose

Legend: PVR, SVR

X-axis: Nitric oxide, 100ng/ml, 300ng/ml, 500ng/ml

Y-axis: 0, -5, -10, -15, -20, -25, -30, -35

Figure 2

**Effect of iv Sildenafil in Anaesthetised Dogs (n=4)**

Figure 3

Effect of Inhaled Sildenafil in Anaesthetised Dogs
(n=3)